# EUROPEAN PATENT APPLICATION

(11) **EP 3 626 207 A1**
(43) Date of publication of application: **25.03.2020**
(21) Application number: 18195749.9
(22) Date of filing: 20.09.2018
(51) Int. Cl.: A61F 2/24

(54) **A MEDICAL DEVICE FOR IMPROVING FUNCTION OF A HEART VALVE**

(71) Applicant: Medtentia International Ltd Oy, 02600 Espoo (FI)
(72) Inventor: KERÄNEN, Olli, 23741 Bjärred (SE)
(74) Representative: Berggren Oy, Helsinki & Oulu

(57) **Abstract**

A medical device (100) for improving function of a heart valve comprises at least a first partly pliant leaflet (101) having a distal section (102) and a proximal section (103). The distal section (102) is configured to be oriented towards said inner section of said valve tissue in use. The first leaflet (101) comprises at least two curving segments, such as bending lines (104, 105) extending from the proximal section (103) towards said distal section (102) thereby dividing said first leaflet to at least three portions (106, 107, 108). The distance (109) between the line ends (104a, 105a) in the distal section (102) area is shorter than the distance (110) between the line ends (104b, 105b) in the proximal section (103) area, thereby allowing at least a distal part of the portion (107a) locating between said curving segments, such as bending lines (104, 105) and being oriented towards said inner section of said valve tissue to bend to a first direction (121) along the curving segments, such as bending lines when in use.

## Description

### TECHNICAL FIELD OF THE INVENTION

The invention relates to a medical device for improving function of a heart valve. Especially the invention relates to a medical device for improving the function of leaflets or replacing the original leaflet or leaflets at least partly.

### BACKGROUND OF THE INVENTION

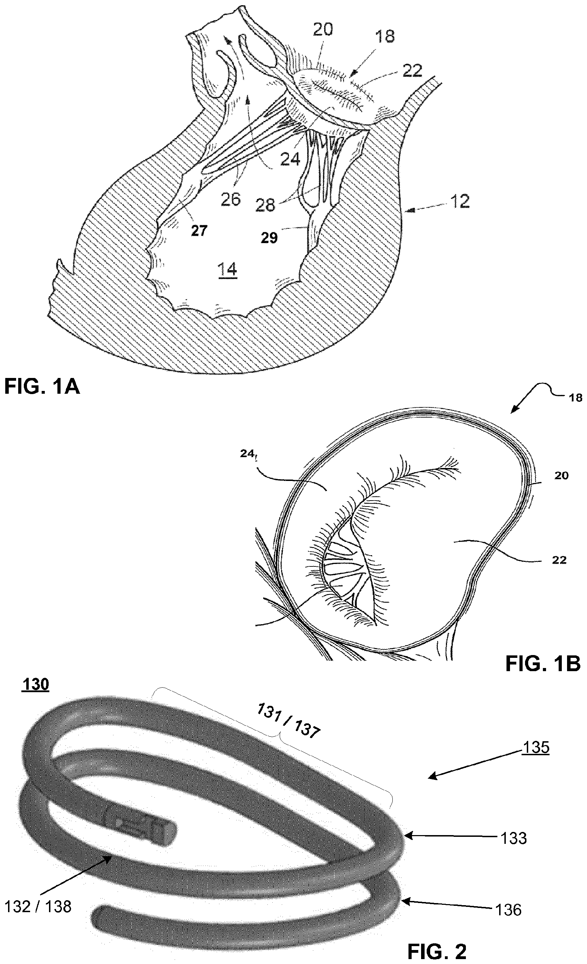

Fig. 1A illustrates a portion of the heart 12, the mitral valve 18, and the left ventricle 14. The mitral valve is at its boundary circumferenced by an annulus 20. The valve has two cusps or leaflets 22, 24. Each of these cusps or leaflets 22, 24 are connected to a respective papillary muscle 27, 29 via their respective connecting chordae 26, 28. In normal healthy individuals the free edges of the opposing leaflets will close the valve by coaptation. However, for some individuals the closure is not complete, which results in a regurgitation, also called valvular insufficiency, i.e. back flow of blood to the left atrium making the heart less effective and with potentially severe consequences for the patient. Fig. 1B illustrates a mitral valve 18, in which the leaflets 22, 24 do not close properly. This commonly occurs when the annulus 20 becomes dilated. One surgical procedure to correct this is to remove a portion of the leaflet 24 and stitch the cut edges together with one another. The procedure will pull back the annulus 20 to a more normal position. However, the strength of the leaflet 24 is altered. Similar problems with a less effective heart function occur if one or both leaflets are perforated to such an extent that blood is flowing towards the left atrium, although the leaflets close properly.

In some conditions of degenerated heart function, the leaflets do not present a solid surface, as in a degenerative valve disease. The leaflet could also be perforated, with one or several holes, where the blood can flow backwards into the atrium. Another possibility is that the leaflet is ruptured, most commonly at an edge of a leaflet, resulting in an incomplete coaptation.

There are artificial leaflets known from the prior art, such as disclosed in EP2591754 (applicant's own publication), where an artificial flexible leaflet reinforcement patch is advantageously connected to an anchoring unit for anchoring the artificial flexible leaflet reinforcement patch to the annulus and additionally for arranging the artificial flexible leaflet reinforcement patch in juxtaposition with the leaflet.

Even if the artificial flexible leaflet reinforcement patch has numerous advantages, there are however still some disadvantages relating to the known prior art artificial leaflets, such as they are difficult or even impossible to deliver to the atrium or ventricle and in connection with the annulus or natural leaflets via a catheter, because the leaflets are often still too rigid and they cannot be bend enough when tried to delivered via the catheter the inner diameter of which is typically only 5-7 mm. In addition, even if the artificial leaflets are pretty much flexible, they should still be flexible advantageously only in one direction (to the direction of normal blood flow) and not flexible to opposite direction. This feature is very hard to achieve by the prior art artificial leaflet patch.

Hence, a medical device for delivering the medical device would be advantageous, and in particular such a device allowing for repair of one or more leaflets of a heart valve, or other related anatomical leaflet structures.

### SUMMARY OF THE INVENTION

An object of the invention is to alleviate and eliminate the problems relating to the known prior art. Especially the object of the invention is to provide a medical device, which is anatomical and flexible in a direction of blood flow in atrium and the flexibility of which is limited to the opposite direction. In addition, the object of the invention is to provide the medical device which is easy to deliver to the atrium via a catheter.

The object of the invention can be achieved by the features of independent claims.

The invention relates to a medical device for improving function of a heart valve according to claim 1.

According to an embodiment of the invention a medical device for improving function of a heart valve is provided. The heart valve comprises valve tissue, including an annulus at an outer section of the valve tissue and a plurality of leaflets at an inner section of said valve tissue, as is depicted already in Figures 1A and 1B. The medical device comprises at least a first partly pliant, so flexible, leaflet or cusp (leaflet hereafter) having a distal section and a proximal section. The first leaflet is advantageously the anterior leaflet, which is also a dominant leaflet for example in a mitral valve. The distal section of the leaflet is configured to be oriented towards the inner section of the valve tissue when in use.

According to an advantageous embodiment of the invention the first leaflet comprises at least two curving segments, such as bending lines extending from the proximal section towards the distal section and thereby dividing the first leaflet to at least three portions. The bending lines, for example, can be provided for example by creasing or bending or folding the leaflet so that to bring the bending lines. Also a groove can be provided for example to the surface of the leaflet or the surface or material can be made thinner and thereby achieve the bending lines or other curving segments. In addition two or more different materials for example with different stiffnesses can be used in the curving segments for dividing the leaflet to two or more areas. The curving segments are advantageously provided so that the area between the curving segments, most advantageously the distal portion of the area between the curving segments, can open or bend into a first direction so in the direction of the blood flow when in use, but so that the curving segments resist the bending of the area to the opposite direction.

Most advantageously the curving segments are provided to the leaflet so that a distance between the line ends in the distal section of the leaflet is shorter than the distance between the curving segment line ends in the proximal section area of the leaflet. This further controls the distal part of the portion locating between said curving segments and being oriented towards the inner section of the valve tissue to bend to the first direction along the curving segments.

According to an exemplary embodiment the medical device may comprise only the first partly pliant leaflet as an anterior leaflet. According to another exemplary embodiment the medical device may comprise only the first partly pliant leaflet as a posterior leaflet.

According to an embodiment the medical device further comprises at least a second partly pliant leaflet, which can be used for example for posterior leaflet. The second leaflet is advantageously the posterior leaflet, which is also a non-dominant leaflet for example in a mitral valve. The second leaflet is advantageously positioned essentially opposite side of the first partly pliant leaflet of the device. The second partly pliant leaflet comprises also a distal section and a proximal section, wherein the distal section is configured to be oriented towards said inner section of said valve tissue and towards the first partly pliant leaflet in the device.

The second pliant leaflet device may, according to an embodiment, also comprise at least two curving segments extending from the proximal section towards the distal section thereby dividing the second leaflet to at least three portions. Most advantageously the curving segments are provided to the second leaflet so that a distance between the bending line ends in the distal section area of the second leaflet is shorter than the distance between the curving segments ends in the proximal section area of the second leaflet. This allows at least a distal part of the portion locating between the curving segments and being oriented towards the inner section of the valve tissue to bend to the first direction along the bending lines, so to the direction of the flow when in use.

Advantageously the first leaflet is a dominant leaflet and it has a first extension between the proximal and distal sections. The second leaflet is a non-dominant leaflet and it has a second extension between the proximal and distal sections, where the second extension is advantageously shorter than said first extension. The first and second leaflets are arranged advantageously so that at least a portion at the distal section of the second leaflet and at least a portion at the distal section of the first leaflet overlap each other to form a coaptation surface area. As an example, the coaptation surface area or height is configured to be formed in a position where a first length of the first leaflet and second length of the second leaflet overlap, wherein the first length is longer than said second length. In some embodiment the first length is about two times said second length from the proximal end. This formulation prevents or at least minimizes effectively the possible backflow of the blood from the ventricle back to atrium. In most cases this corresponds about 7-9 mm coaptation. In addition it is to be noted that the overlap range depends on case and may differ, and in addition it is to be noted that the first and/or second leaflets may extend also beyond said coaptation surface area towards the left ventricle. Advantageously the first leaflet can extend farther that se second one.

According to an exemplary embodiment the first extension is about 2 times longer than the second extension. In addition, the distal section of the second leaflet is configured to locate on the top of the first leaflet (in the direction of the flow) so to allowing at least the distal part of the portion of the first leaflet to bend to the first direction.

The leaflets may comprise for example leather or tissue, such as pericardium tissue (for example from an animal, such as a sheep, pig or horse), or artificial tissue (scaffold) or fabric, such as polyester. According to an embodiment the flexible leaflet may comprise different materials, such as PTFE or polyester or coretex, or porosity material or fabric having endothelial surface or mesh like surface thereby enabling the natural leaflet to grow into the leaflet of the invention where the flexible leaflet is in juxtaposition against the natural leaflet. This may give a permanent reinforcement of the leaflet area. Furthermore, some embodiments have its portion made of a biocompatible material. As such the medical device will not interfere with the valve tissue, avoiding rejection reactions, and/or avoids causing of blood clotting, embolies, blood cavitations, or also turbulences in the blood flow passing the heart valve.

According to an embodiment the first (and/or second) leaflet may have at least two materials with different elastic properties in order to allow for example the distal section of the leaflet to bend easier than the proximal section of the leaflet. In addition or alternatively also the first and second leaflets may comprise different material, such as the first leaflet a first material and the second leaflet a second material, so that for example the first leaflet, and especially the distal section of the first leaflet, bend easier than the second leaflet, and especially the distal section of the second leaflet. This may further enhance the functioning of the leaflets.

According to an embodiment the distal ends of the first and second leaflet can be fastened to each other at least at certain portions. The fastening can be implemented by a clip or suture, for example. By this the blood flow and circulation from the atrium to the ventricle can be controlled, as well as to decrease the regurgitation by 1-2 degree of 4.

In addition, according to an advantageous embodiment the medical device comprises a tissue anchoring unit for anchoring the leaflets to the tissue of the heart valve, such as to the annulus, for example. The tissue anchoring unit functions as a body structure, to which said leaflets are fastened. In addition the diameter of the annulus can also be downsized by the tissue anchoring unit so that the diameter of the annulus is at first downsized and after this the tissue anchoring unit is fastened to the downsized annulus. The tissue anchoring unit comprises advantageously first and second sections and it is configured to abut a first (or in some embodiment a second) side of the heart valve. The first leaflet is advantageously fastened to the first section of the tissue anchoring unit, advantageously via the proximal section of the first leaflet. In addition, the second partly flexible leaflet is advantageously fastened to the second section of the tissue anchoring unit, advantageously via the proximal section of the second leaflet.

However, it is to be noted that according to an embodiment the size of at least one or both leaflets can be so big that the downsizing is not needed but the distal sections of the leaflets can overlap each other and form the coaptation surface.

The tissue anchoring unit, when in use, secures the leaflet(s) to the adjacent tissue and/or annulus, in such a manner that the leaflet(s) is(are) prevented from dislocating itself, without loss of the desired function of the leaflets. Hence, the leaflet(s) will be less affected by the forces of the pulsatile blood flow and the dynamics of the beating heart, which would dislocate the leaflet(s) otherwise.

The tissue anchoring unit for some embodiments is or forms a loop-shaped support. This gives a more rigid construction that abuts against the whole extension of adjacent valve tissue, e.g. the annulus. In addition the medical device is easier to handle by the operator during operation or intervention.

Some embodiments have a tissue anchoring unit which has fastening units as glue, spikes, prongs, points, hooks, clasps or hasps. These fastenings units further secure attachment of the medical device to adjacent valve tissue, e.g. annulus. The fastening units are in some embodiments made of a biocompatible material. In other embodiments these fastening units are made of biodegradable material or bioabsorbable material. The latter embodiments act to reinforce the leaflets during a restricted period of time, as and when required.

The embodiment described offer many advantages over the known prior art, such as providing anatomical medical device, which is flexible in a direction of the blood flown in atrium and non-flexible to the opposite direction from its normal or rest state, where no external forces are applied. In particularly this means that the leaflets are configured to open or bend or be flexible towards the ventricle and closed or non-bendable or non-flexible towards the atrium essentially from the level of the annulus of the mitral valve. In addition, the object of the invention is to provide the medical device which can be delivered to the atrium and again to be introduced with the annulus via a catheter.

Moreover, the embodiment described allows the first leaflet to imitate the function of the natural anterior dominant leaflet and the second leaflet to imitate the function as the posterior non-dominant leaflet as far as possible. This is achieved by the dimensions, arrangement, overlapping and curving segments, such as bending lines described in the embodiments. The invention allows at least the distal part of the first pliant leaflet of the first and second pliant leaflets to bend to the first direction (in the direction of the blood flow and towards the ventricle) in that way that, when in use, the first leaflet guides the blood flow to head towards the posterior wall of the left ventricle in a very natural way. In this way the blood flow smoothly follows the curvature lines of the wall shape of the left ventricle and the flow is as laminar as possible and flows towards aorta outflow track. Due to the laminar blood flow in the left ventricle, the flow circulation is very effective with negligible resistance or loss, providing good blood flush. In addition, due to proper laminar blood flow, possible blood clotting, embolies, blood cavitations, or turbulences in the blood flow passing the heart valve can be avoided. These advantages are not possible for example with the leaflets, the distal portions of which do not bend properly, not with the leaflets of same size.

Still in addition the medical device having the tissue anchoring unit with the first and second loop-shaped support structures and the leaflets fastened via their proximal portions to the tissue anchoring unit has a further advantage, namely when the tissue anchoring unit with the leaflets is secured to the tissue and/or the annulus, the second and the first support structures will thereby trap a portion of the valve tissue between them and additionally secure and block any possible leakage between the ventricle and atrium.

The exemplary embodiments presented in this text are not to be interpreted to pose limitations to the applicability of the appended claims. The verb "to comprise" is used in this text as an open limitation that does not exclude the existence of also unrecited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:
- Figures 1A-1B: illustrate schematically a portion of a heart and mitral valve,
- Figure 2: illustrates an exemplary tissue anchoring unit for securing the flexible leaflet to the adjacent tissue and/or annulus according to an advantageous embodiment of the invention,
- Figure 3: illustrates an example of a first flexible leaflet according to an advantageous embodiment of the invention,
- Figure 4: illustrates an example of a second flexible leaflet according to an advantageous embodiment of the invention,
- Figure 5: illustrates an exemplary medical device having the first and second flexible leaflets in a delivery state according to an advantageous embodiment of the invention, and
- Figures 6A-6B: illustrate an exemplary medical device having the first and second flexible leaflets secured to the annulus by the tissue anchoring unit according to an advantageous embodiment of the invention.

### DETAILED DESCRIPTION

Figures 1A-1B illustrating schematically a portion of a heart and mitral valve are already discloses in the connection with the background portion of the invention.

Figure 2 illustrates an exemplary tissue anchoring unit 130 for securing the flexible leaflets 101, 111 (not shown in Figure 2) to the adjacent tissue and/or annulus 20 according configured to abut a first side of the heart valve and configured to abut a first side of the heart valve. The tissue anchoring unit 130 comprises a first loop-shaped support structure 133. The first loop-shaped support structure 133 comprises first and second sections 131, 132, and the first and second sections 131, 132 are arranged to locate (in use so in an activated state) essentially at the opposite sides of the loop-shaped support structure 133 when the tissue anchoring unit 130 takes the loop formation in an activated state.

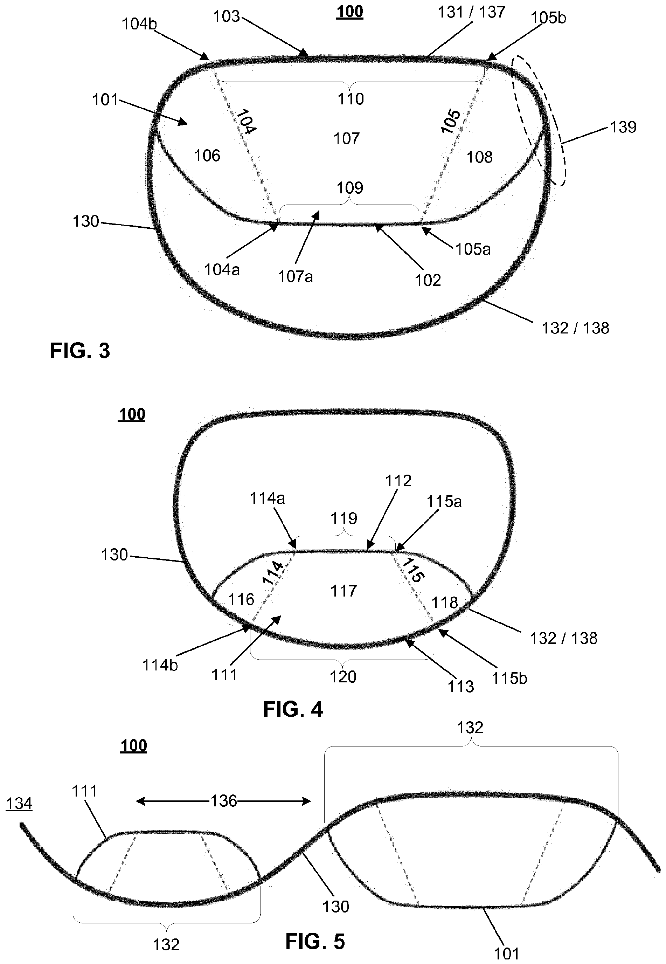

The first loop-shaped support structure 133 is advantageously anatomical D-shape or anatomical kidney shape having essentially an elongated portion 137 and a curved portion 138. The elongated portion 137 is configured to be faced to the anterior side of the mitral valve 18 and the curved portion 138 to the posterior side of the mitral valve 18, when in use. The first section 131 of the tissue anchoring unit 130 is located essentially at the elongated portion 137 to which the first partly flexible leaflet 101 is fastened via the proximal section 103 of the first partly flexible leaflet 101 (as shown in Figures 3 and 5). In addition, according to an exemplary and optional embodiment, the second partly flexible leaflet 111 is fastened to the second section 132 (so to the curved portion 138) of the tissue anchoring unit 130 advantageously via the proximal section 112 of the second partly flexible leaflet patch 111 (as shown in Figures 4 and 5A-5B).

Advantageously the tissue anchoring unit 130 comprises additionally also a second loop-shaped support structure 136 forming with the first loop-shaped support structure a continuous helix structure, when being in activated state 135 (as is depicted in Figure 2). The second loop-shaped support structure is configured to abut a second, opposite, side of the valve to thereby trap a portion of the valve tissue 20 between the second and the first support structures 133, 136.

The tissue anchoring unit 130 has also a delivery state 134 (depicted in Figure 5), where the tissue anchoring unit 130 has an elongated form so that said first and second sections 131, 132 locate essentially successively in a longitudinal direction 136 of the elongated form. In said delivery state the medical device can be transferred advantageously through a catheter having diameter 5-7 mm, for example.

The tissue anchoring unit advantageously comprises a shape memory material having a first shape, such as the elongated form of the delivery state in a first temperature, and the second shape, such as the loop-shaped form in a second temperature. The second temperature corresponds advantageously essentially the body temperature, whereupon the tissue anchoring unit takes the second shape, so the loop-shaped form, when introduced for example with the blood flow in the atrium.

As is illustrated the first and/or second leaflets can be arranged to the first loop-shaped support structure 133. However, even if not depicted in Figures, the first and/or second leaflets can also be arranged to the corresponding portions of the second loop-shaped support structure 136, which can offer advantages such that the distal portions of the first and second leaflets will be pressed against each other more tightly and thereby prevent the backflow more effectively during the left ventricle contract. However, sometimes the chordae might be problematic if the leaflets are arranged to the second loop-shaped support structure 136, which in turn is not problem when the leaflets are arranged to the first loop-shaped support structure 133.

Figure 3 illustrates an example of a medical device 100 having a first flexible leaflet 101 and Figure 4 a second flexible leaflet 111 according to an advantageous embodiment of the invention. The first (anterior, dominant) leaflet 101 has a distal section 102 and a proximal section 103, where the distal section 102 is configured to be oriented towards the inner section of the valve tissue when in use. The first leaflet 101 comprises at least two bending lines 104, 105 (as an example of the curving segments) extending from the proximal section 103 towards the distal section 102, thereby dividing the first leaflet to at least three portions 106, 107, 108. The bending lines 104, 105 can be provided for example by creasing or bending or folding the leaflet, or by some other way known by the skilled person. The bending lines are advantageously provided so that the area 107 between the bending lines, most advantageously the distal portion 107a of the area 107 between the bending lines, can open or bend only into a first direction 121 from its normal state (when no external force is applied) so in the direction of the flow when in use, but essentially not to the opposite direction thereby preventing any backflow from the left ventricle back to the atrium.

The distance 109 between the line ends in the distal section 102 is advantageously shorter than the distance 110 between the bending line ends in the proximal section 103 area of the leaflet. The first partly flexible leaflet 101 is fastened to the first section 131 of the tissue anchoring unit 130 advantageously via the proximal section 103 of the first partly flexible leaflet 101.

The end of at least one of the two bending lines 104, 105 in the proximal section 103 of the first partly flexible leaflet 101 origins or extends advantageously and essentially from the portion 139 of the tissue anchoring unit 130 where the elongated portion 137 and curved portion 138 join together. This allows very advantageous and proper bending to at least for the distal part of the portion 107a of the first leaflet 101.

The exemplary second flexible leaflet 111 comprises advantageously also at least two bending lines 114, 115 extending from the proximal section 113 towards the distal section 112 thereby dividing the second leaflet 111 to at least three portions 116, 117, 118. Most advantageously the bending lines 114, 115 are provided to the second leaflet so that a distance 119 between the bending line ends in the distal section 112 area of the second leaflet is shorter than the distance 120 between the bending line ends in the proximal section 112 area of the second leaflet.

The second leaflet 111 is advantageously fastened to the second section 132 of the tissue anchoring unit 130, advantageously via the proximal section 112 of the second leaflet 111. Advantageously the second leaflet 111 is positioned essentially opposite side of the first partly flexible leaflet 101 in the tissue anchoring unit 130 of the device 100.

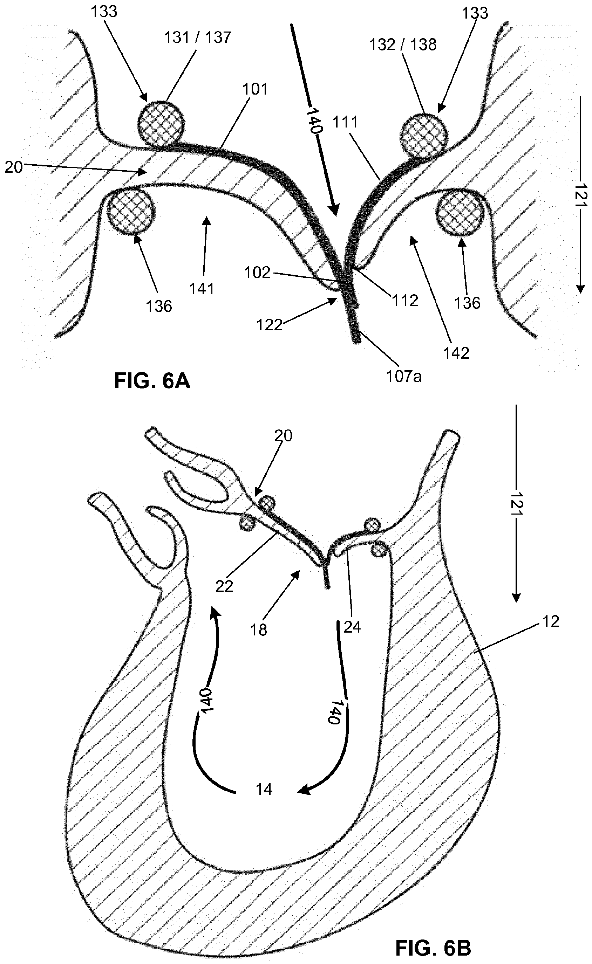

Figure 5 illustrates an exemplary medical device 100 having the first and second flexible leaflets 101, 111 in a delivery state 134 according to an advantageous embodiment of the invention. In said delivery state 134 the tissue anchoring unit 130 has an elongated form so that said first and second sections 131, 132 locate essentially successively in a longitudinal direction 136 of the elongated form. As described elsewhere in this document, the tissue anchoring unit 130 advantageously comprises a shape memory material having a first shape, such as the elongated form of the delivery state 134 (illustrated in Figure 5) in a first temperature. When the tissue anchoring unit 130 is exposed to the second temperature, for example when introduced with the blood flow in the atrium, it will take the second shape, such as the loop-shaped form depicted in Figures 2 and 6A, 6B (in use).

In said loop-shaped form of the tissue anchoring unit 130, so in use, the first and second sections 131, 132 are configured to turn or twist from the delivery state so to the said first and second sections 131, 132 will be located essentially at the opposite sides of the loop-shaped support structure 133. In said loop-shaped form the tissue anchoring unit 130 can be secured to the annulus 20, as is depicted in Figures 6A and 6B.

Figures 6A-6B illustrate an exemplary medical device 100 having the first and second flexible leaflets 101, 111 secured to the annulus 20 by the tissue anchoring unit 130 according to an advantageous embodiment of the invention.

The first leaflet 101 is typically bigger (the extension or length is greater) than the second leaflet 111 so that the first leaflet 101 has the first extension, whereas the second leaflet 111 has the second extension. Therefore the first leaflet 101 is used advantageously as a dominant leaflet and it is configured to be arranged in the anterior side 141. The second leaflet 111 is a non-dominant leaflet it is configured to be arranged in the posterior side 142.

The first and second leaflets 101, 111 are arranged advantageously so that at least the distal section 112 of the second partly pliant leaflet 111 and at least the distal section 102 of the first partly pliant leaflet 101 overlap each other to form a coaptation surface area 122. The first and second leaflets 101, 111 may be configured for example to overlap so that a first length of the first leaflet 101 is faced and a second length of the second leaflet 111 are faced towards the left atrium before overlapping each other at the coaptation surface area. The first length is advantageously about two times greater than said second length.. In addition, the first and/or second leaflets 101, 111 advantageously extend also beyond the coaptation surface area 122 towards the left ventricle 14. This effectively ensures the coaptation surface area 122 to be sufficient. In most cases this corresponds about 7-9 mm coaptation.

As can be seen in Figures 6A-6B the distal section 112 of the second leaflet 111 is advantageously configured to locate on the top of the first leaflet 101 in the direction of the flow 140 so to allowing at least the distal part of the portion 107a of the first leaflet to bend to the first direction 121. This is advantageous because the first leaflet 101 is the dominant and must be clear for opening in the direction of the flow 121.

In the exemplary Figures 6A-6B the tissue anchoring unit 130 comprises both the first loop-shaped support structure 133 and the second loop-shaped support structure 136 forming with the first loop-shaped support structure as a continuous helix structure so to abut a second, opposite, side of the valve and thereby to trap a portion of the valve tissue 20 between the second and the first support structures 133, 136. However, even if the both first and second loop-shaped support structure are shown in Figures 6A-6B, it should be noted that the tissue anchoring unit 130 having only the first loop-shaped support structure 133 can also be applied.

In addition it is to be noted that the first and second loop-shaped support structures 136 may be made of same structure or material so that the tissue anchoring unit 130 having both first and second loop-shaped support structures is as a continuous unit or helix structure. Alternative the first and second loop-shaped support structures may be two separate portions, which are physically connected to each other and thereby form said helix structure.

The elongated portion 137 and thus also the first section 131 (and the first leaflet 101 fastened to said first section 131) of the tissue anchoring unit 130 is configured to be faced to the anterior side 141 of the mitral valve 18. The curved portion 138 and thus also the second section 132 (and the second leaflet 111 fastened to said second section 132) of the tissue anchoring unit 130 is configured to be faced to the posterior side 142 of the mitral valve 18. In use, so when the tissue anchoring unit 130 has the loop-shaped form, the first leaflet 101 locates essentially opposite to the second leaflet 111.

The invention has been explained above with reference to the aforementioned embodiments, and several advantages of the invention have been demonstrated. It is clear that the invention is not only restricted to these embodiments, but comprises all possible embodiments within the spirit and scope of the inventive thought and the following patent claims.

The features recited in dependent claims are mutually freely combinable unless otherwise explicitly stated. For example, it is to be noted that the bending lines in the second leaflet is an optional feature and that the second leaflet can be provided also to the medical device of the invention without any bending lines. This is possible because the first leaflet having the bending lines, is a dominant leaflet. In addition it is to be noted that even if only two bending lines are described in the embodiments, also more than two bending lines can be provided.

In addition it is to be noted that the bending lines illustrated and depicted in Figures and description above are examples of the curving segments, but naturally the curving segments can be implemented also in other ways described elsewhere in this document.

## Claims

1. A medical device (100) for improving function of a heart valve, the heart valve comprising valve tissue, including an annulus at an outer section of said valve tissue and a plurality of leaflets (24) at an inner section of said valve tissue,
wherein the medical device comprises:
- at least a first partly pliant leaflet (101) (=anterior) having a distal section (102) and a proximal section (103), wherein said distal section (102) is configured to be oriented towards said inner section of said valve tissue, and
- said first leaflet (101) comprising at least two curving segments, such as bending lines, (104, 105) extending from the proximal section (103) towards said distal section (102) thereby dividing said first leaflet to at least three portions (106, 107, 108) so that the distance (109) between the line ends (104a, 105a) of the curving segments (104, 105) in the distal section (102) area is shorter than the distance (110) between the line ends (104b, 105b) in the proximal section (103) area and thereby allowing at least a distal part of the portion (107a) locating between said curving segments (104, 105) and being oriented towards said inner section of said valve tissue to bend to a first direction (121).

2. A medical device of claim 1, wherein the medical device further comprises:
- at least a second partly pliant leaflet (111) positioned essentially opposite said first partly flexible leaflet (101), said second partly flexible leaflet (111) having a distal section (112) and a proximal section (113), wherein said distal section (112) is configured to be oriented towards said inner section of said valve tissue and said first partly flexible leaflet patch (101).

3. A medical device of claim 2, wherein said second leaflet (111) comprises at least two curving segments, such as bending lines, (114, 115) extending from the proximal section (113) towards said distal section (112) thereby dividing said second leaflet (111) to at least three portions (116, 117, 118) so that the distance (119) between the line ends (114a, 115a) in the distal section (112) area is shorter than the distance (120) between the line ends (114a, 115a) in the proximal section (112) area and thereby allowing at least a distal part of the portion locating between said curving segments, such as bending lines and being oriented towards said inner section of said valve tissue to bend to a first direction along the bending lines.

4. A medical device of any previous claims 2-3, wherein at least the distal section (112) of the second partly pliant leaflet (111) and at least the distal section (102) of the first partly pliant leaflet (101) are configured to overlap each other to form a coaptation surface area (122).

5. A medical device of claim 4, wherein the coaptation surface area (122) is configured to be formed in a position where a first length of the first leaflet (101) and second length of the second leaflet (101) overlap, wherein the first length is longer than said second length and advantageously about two times said second length.

6. A medical device of any previous claims 2-5, wherein at least a distal part of said first and second partly flexible leaflets (101, 102) is configured to bend to a first direction (121).

7. A medical device of any previous claims, wherein the medical device comprises a tissue anchoring unit (130) having first or anterior and second or posterior sections (131, 132) and configured to abut a first side of the heart valve, wherein the first partly flexible leaflet (101) is fastened to the first section (131) of the tissue anchoring unit (130).

8. A medical device of claim 7, wherein the second partly flexible leaflet (111) is fastened to the second section (132) of the tissue anchoring unit (130).

9. A medical device of any claims 7-8, wherein the tissue anchoring unit (130) comprises a first loop-shaped support structure (133) having said first and second sections (131, 132), where said first and second sections (131, 132) are arranged to locate essentially at the opposite sides of the loop-shaped support structure (133) when the tissue anchoring unit (130) is in the loop formation.

10. A medical device of any of claims 7-9, wherein the tissue anchoring unit (130) has a delivery state (134) and an activated state (135), wherein
- in said delivery state (134) the tissue anchoring unit (130) has an elongated form so that said first and second sections (131, 132) locate essentially successively in a longitudinal direction (136) of said elongated form,
- wherein in the activated state the tissue anchoring unit (130) has said loop-shaped formation and
- wherein the tissue anchoring unit (130) is transferable from said delivery state (134) to said activated state (135).

11. A medical device of any of claims 7-10, wherein the tissue anchoring unit (130) comprises additionally a second loop-shaped support structure (136) configured to abut a second, opposite, side of the valve to thereby trap a portion of the valve tissue (20) between the second and the first support structures (133, 136).

12. A medical device of any of claims 7-11, wherein the tissue anchoring unit (130) comprises a first loop-shaped support structure (133) having essentially a D-shape form having essentially an elongated portion (137) and a curved portion (138), wherein the first section (131) of the tissue anchoring unit (130) locates essentially at said elongated portion (137) and whereupon the first partly flexible leaflet (101) is fastened to said first section (131) of the tissue anchoring unit (130) advantageously via the proximal section (103) of the first partly flexible leaflet (101).

13. A medical device of claim 12, wherein the end of at least one of said two curving segments, such as bending lines (104, 105) in the proximal section (103) of the first partly flexible leaflet (101) origins or extends essentially from the portion of the tissue anchoring unit (130) where the elongated portion (137) and curved portion (138) join together.

14. A medical device of any of claims 7-13, wherein the tissue anchoring unit (130) comprises a first loop-shaped support structure (133) having essentially a D-shape form having essentially an elongated portion (137) and a curved portion (138), wherein the second section (132) of the tissue anchoring unit (130) locates essentially at said curved portion (138) and whereupon the second partly flexible leaflet (111) is fastened to said second section (132) of the tissue anchoring unit (130) advantageously via the proximal section (113) of the second partly flexible leaflet (111).

15. A medical device of any of previous claim, wherein the first and/or second partly flexible leaflet (101, 111) comprises leather, tissue, such as pericardium tissue or artificial tissue, fabric, such as polyester or coretex, or porosity material or fabric supporting endothelial surface growth or mesh like surface thereby enabling the leaflet to grow into a natural leaflet in use where the flexible leaflet is in juxtaposition against the natural leaflet.

16. A medical device of any of previous claim, wherein said curving segments, such as bending lines (104, 105, 114, 115) are configured to allowing at least a distal part (107, 117) of the portion locating between said curving segments, such as bending lines and being oriented towards said inner section of said valve tissue to bend only to said first direction (121) along the bending lines and preventing to bend to the opposite direction.

17. A medical device of any of previous claim, wherein at least a part of the proximal portion (103, 113) of the flexible leaflet (101, 111) is configured to be in juxtaposition to at least one leaflet in use.
